(19) Europäisches Patentamt
European Patent Office
Office européen des brevets

(11) Numéro de publication: **0 187 076 B1**

(12) **FASCICULE DE BREVET EUROPEEN**

(45) Date de publication de fascicule du brevet: **18.09.91**

(51) Int. Cl.5: **G01N 33/53**, G01N 33/533

(21) Numéro de dépôt: **85402360.3**

(22) Date de dépôt: **29.11.85**

Le dossier contient des informations techniques présentées postérieurement au dépôt de la demande et ne figurant pas dans le présent fascicule.

(54) **Composés fluorescents, dérivés d'(amino-4) naphtalimide, leur application aux dosages immunologiques en polarisation de fluorescence.**

(30) Priorité: **30.11.84 FR 8418311**

(43) Date de publication de la demande: **09.07.86 Bulletin 86/28**

(45) Mention de la délivrance du brevet: **18.09.91 Bulletin 91/38**

(84) Etats contractants désignés: **DE FR GB NL**

(56) Documents cités:
**FR-A- 2 518 096**
**US-A- 4 473 693**

**NATURE, vol.292, no.5818, 02 juillet 1981, MacMillan Journals Ltd., Chesham, Bucks (GB); W.W.STEWART, pp.17-21**

**CHEMICAL ABSTRACTS, vol.99, no.13, 26 septembre 1983, Columbus, OH (US); P.BAILEY et al., p.303, no.101708m**

(73) Titulaire: **Etablissement Public dit: CENTRE NATIONAL DE LA RECHERCHE SCIENTIFIQUE (CNRS)**
**15, Quai Anatole France**
**F-75007 Paris(FR)**

(72) Inventeur: **Cittanova, Nicole**
**147 boulevard Raspail**
**F-75006 Paris(FR)**
Inventeur: **Desfosses, Bernard**
**11 rue de la Montagne Ste Geneviève**
**F-75005 Paris(FR)**
Inventeur: **Christeff, Nicolas**
**120 rue d'Assas**
**F-75006 Paris(FR)**
Inventeur: **Rajkowski, Krzysztof**
**159 rue de Charonne**
**F-75011 Paris(FR)**

(74) Mandataire: **Phélip, Bruno et al**
**c/o Cabinet Harlé & Phélip 21, rue de La Rochefoucauld**
**F-75009 Paris(FR)**

**EP 0 187 076 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

JOURNAL OF THE AMERICAN CHEMICAL SOCIETY, vol.103, no.25, 1981, American Chemical Society (US); W.W.STEWART, pp.7615-7620

CHEMICAL ABSTRACTS, vol.52, no.4, 25 février 1957, Columbus, OH (US); B.F.ERLANGER et al., no.2979i

## Description

La présente invention concerne de nouveaux réactifs fluorescents, dérivés d'(amino-4) naphtalimide , et l'application de ces réactifs au dosage de composés susceptibles de se coupler à des anticorps, utilisant une mesure de polarisation de fluorescence.

On sait qu'un composé fluorescent de petite masse moléculaire présente en solution, un taux de polarisation faible, c'est-à-dire que la lumière fluorescente qu'il émet lorsqu'il est soumis à une lumière polarisée linéairement, est fortement dépolarisée, alors qu'un composé fluorescent de grand volume moléculaire, dont le mouvement propre en solution est beaucoup plus lent, aura un taux de polarisation nettement plus élevé. Ce principe a été mis à profit pour doser, dans les liquides biologiques, des antigènes ou des haptènes selon deux procédés, le premier notamment décrit dans la demande de brevet FR-A-2 500 165, déposée par Abbott Laboratories, le 16 Février 1982, le deuxième basé sur le marquage des fragments Fab d'une immunoglobuline monoclonale par un composé fluorescent. Dans le premier procédé, on met en oeuvre un composé fluorescent de petite masse moléculaire qui résulte de la fixation par une liaison irréversible, sur une dicarboxyfluoresceïne d'un haptène ou antigène à doser, qui doit comporter une fonction amine pour former une liaison -CONH- avec l'un des carboxyles du marqueur fluorescent. Lorsqu'on introduit , dans le milieu où se trouve l'haptène ou antigène à doser, désigné dans ce qui suit par Ag, une quantité connue du composé fluorescent préparé à partir du produit à doser et de la dicarboxyfluoresceïne, ainsi qu'une quantité donnée d'anticorps Ac dirigés contre Ag, il y a compétition entre les molécules du composé Ag à doser et celles du composé fluorescent qui contient ce même Ag pour la fixation sur l'anticorps Ac; cette compétition n'a évidemment lieu que dans la mesure où Ac est présent en quantité insuffisante pour que toutes les molécules d'Ag à doser et celles du composé fluorescent donnent lieu à la formation d'un complexe avec Ac; la quantité de complexe formé entre Ac et le composé fluorescent est alors directement fonction de la quantité d'Ag se trouvant dans le milieu à étudier.Comme par ailleurs, le composé fluorescent libre présente un faible taux de polarisation de fluorescence P, alors que le complexe : composé fluorescent/anticorps a un P élevé , le procédé de dosage décrit dans FR-A-2 500 165 consiste à mesurer la différence des taux de polarisation des milieux contenant l'Ag à doser et le composé fluorescent correspondant en l'absence et en présence d'Ac, et à en déduire à l'aide de courbes d'étalonnage la concentration d'Ag initialement présente dans le milieu.

La demande de brevet FR-A-2.518.096 déposée comme la précédente par Abbott Laboratories a pour objet un procédé de dosage basé sur le même principe et utilisant comme marqueurs des dérivés d'aminofluorescéïne .

Le deuxième procédé est basé sur l'utilisation d'une ou plusieurs molécules d'un composé fluorescent fixées covalemment aux fonctions amine, alcool, thiol ou carboxylique des fragments Fab purifiés d'immunoglobulines monoclonales dirigés contre un antigène de haut poids moléculaire . Dans ce qui suit ,nous désignerons ces fragments Fab fluorescents par FabF et l'antigène par Ag. Lorsqu'on introduit dans le milieu où se trouve l'Ag à doser une quantité connue de FabF, il y a fixation d'Ag sur les sites de reconnaissance de FabF.Le composé FabF possède un taux de polarisation P relativement faible alors que le complexe FabF.Ag a un P élevé. Le procédé de dosage consiste donc à mesurer l'augmentation du taux de polarisation de fluorescence P de FabF en présence de l'Ag à doser,cette augmentation étant fonction de la proportion de FabF complexe avec Ag et donc fonction de la concentration de Ag à doser dans le milieu.

La mise en oeuvre de la dicarboxyfluoresceïne ,pour la réalisation du premier procédé de dosage antérieurement décrit, présente divers inconvénients ; avant tout , Ag doit avoir une fonction amine. Ensuite,les composés fluorescents résultants sont sensibles au pH du milieu;non seulement, ils ne sont solubles que dans une gamme de pH limitée, lorsque les groupes carboxyliques sont salifiés, mais surtout l'intensité de l'émission de fluorescence varie avec le pH,ce qui peut nécessiter un réglage de l'appareil de mesure pour chaque échantillon . De plus, la longueur d'onde d'excitation de ces composés fluorescents est assez proche de celle du rayonnement émis , de telle sorte qu'il est difficile de séparer optiquement, c'est-à-dire par des filtres, la lumière émise à mesurer ,de la lumière incidente,partiellement polarisée par diffusion; ceci a pour conséquence une concentration minimale décelable d'Ag relativement importante ,et une diminution de la sensibilité de la méthode .

La présente invention n'a pas ces inconvénients et apporte donc une solution unique à différents problèmes dont l'utilisateur ne décelait pas forcément l'origine.L'invention concerne des procédés de dosage utilisant comme réactifs de dosage des composés fluorescents solubles en milieu aqueux comportant dans leur molécule un groupe fluorescent qui est un (amino-4) naphtalimide substitué sur l'azote de la fonction imide; les composés selon l'invention répondent à la formule I :

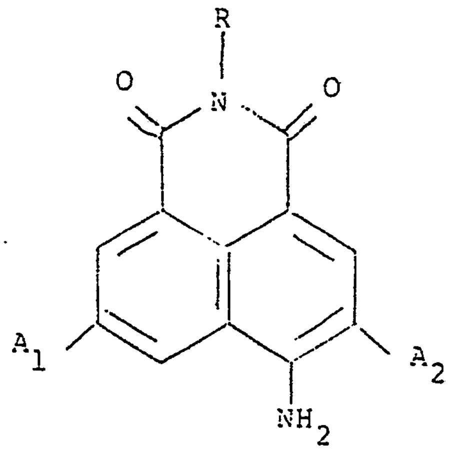

I

dans laquelle $A_1$ et $A_2$, identiques ou différents, représentent l'atome d'hydrogène ou un groupe $SO_3^{\ominus}$ $M^{\oplus}$, $M^{\oplus}$ est un cation métallique, de préférence alcalin, comme le lithium, ou un ammonium, tel que celui dérivé de la diéthanolamine, et R représente, avec l'atome d'azote auquel il est lié, un haptène, antigène ou fragment d'anticorps Fab. Pour les composés fluorescents de formule I concernant l'haptène ou l'antigène, les déterminants antigéniques n'ont pas été modifiés par la réaction chimique. Pour les composés fluorescents de formule I concernant le fragment d'anticorps Fab, le site de reconnaissance de l'antigène n'a pas été modifié par la réaction chimique. En général, l'haptène, l'antigène de masse moléculaire inférieure à 20 000 ou le fragment d'anticorps Fab, n'est pas directement fixé sur l'azote de l'imide mais sur un groupe Z substituant cet atome, dont la structure dépend de la nature des fonctions réactives du composé à greffer. Le groupe Z est choisi de telle sorte que l'imide soit stable même en solution aqueuse et quel que soit le pH, et que les propriétés optiques du composé de formule I (rendement quantique, longueurs d'onde d'émission et d'excitation...) permettent son utilisation dans le dosage de faibles quantités de produits, qui peuvent se trouver dans des milieux divers, notamment dans les liquides biologiques.

Comme on l'a vu précédemment, il est avantageux que la longueur d'onde d'excitation de la molécule soit nettement différente de celle de la lumière de fluorescence émise, que ces longueurs d'onde soient éloignées de celles des rayonnements absorbés par les milieux biologiques, et aussi que le rendement quantique de fluorescence soit élevé, c'est-à-dire supérieur à 0,2 environ.

Parmi les groupes Z préférés, on peut citer:

- le groupe $Z_1$ :

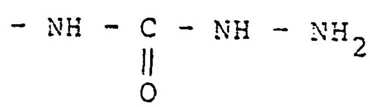

sur lequel des groupes carbonyle ou carboxyle peuvent réagir en donnant des semi-carbazones

- le groupe $Z_2$ :

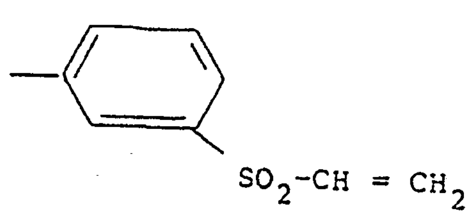

sur lequel dans des conditions douces, se fixeront au niveau de la double liaison éthylénique des amines, thiols, méthylènes activés ou même alcools.

Certains des Ag à greffer peuvent ne comporter aucune des fonctions susceptibles de réagir sur Z dans des conditions n'altérant pas la structure de la région de la molécule cause de la fluorescence du

composé de formule II:

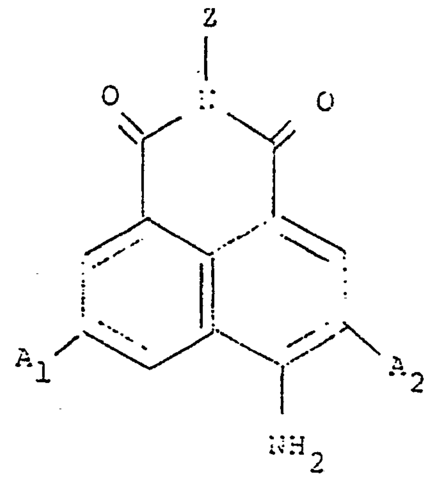

II

Dans ce cas, on peut fixer, selon l'invention, préalablement, sur l'haptène, l'antigène ou le groupe Z , un groupe réactif comportant une fonction réactive convenable ; ainsi lorsque Ag porte un groupe acide carboxylique ou un groupe réactif dérivé, on utilisera avantageusement une diamine aliphatique tel que le diamino-1,5 pentane ou le diamino-1,7 heptane, comme réactif de couplage des groupes $Z_2$ et Ag; lorsque Ag porte des halogènes mobiles ou des groupes époxy ou carboxyliques , on pourra utiliser la cystéamine comme réactif de couplage.L'invention a de plus spécifiquement pour objet de tels réactifs.

Lorsque Ag est porteur d'une fonction amine primaire ou secondaire, aromatique ou aliphatique , le composé selon l'invention peut résulter de l'action de Ag sur l'anhydride de formule III :

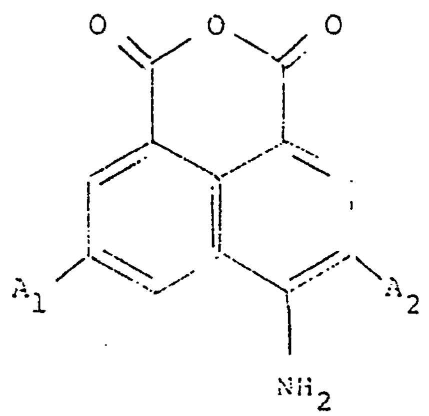

III

dans laquelle $A_1$ et $A_2$ ont les mêmes significations que précédemment , auquel cas l'atome d'azote de l'imide fluorescent de formule I provient de l'haptène ou de l'antigène.

Si Ag à doser ne porte pas de fonction amine convenable,on peut greffer sur Ag un groupe portant une fonction amine comme le diaminoheptane, le diaminopentane ou la cystéamine, en faisant en sorte que les déterminants antigéniques de Ag ne soient pas modifiés, et préparer, en faisant réagir ce nouveau composé sur l'anhydride III,un composé I, utile au dosage de Ag.

Si Ag à doser ne porte pas de fonction amine convenable , l'utilisation de ces divers réactifs de couplage: diaminoheptane et analogues ou cystéamine comme espaceur,est aussi avantageuse lorsque Ag, bien que possédant une fonction qui autorise sa liaison aux composés fluorescents de départ de formule II ou III, a son déterminant antigénique correspondant au Ac utilisé dans le procédé, situé de telle sorte que Ac ne se couple plus à Ag lorsqu'il a été fixé sur un composé de formule II ou III,notamment pour une question d'empêchement stérique.

Si Ag à doser porte une fonction amine convenable , l'acide gamma-aminobutyrique est utilisé comme espaceur .L'invention a aussi pour objet de tels composés .

Les composés de formule II dans laquelle Z est $Z_1$ ou $Z_2$ et $A_1$ et $A_2$ sont $SO_3$ $M^+$ sont décrits par W.W. Stewart (J. Am. Chem. Soc. 1981 Vol.103 n° 25, brevet US-4.473.693, Nature 1981 Vol.292 n° 5818 ) et par M.P. Bailey et al. (Chemical Abstracts, Vol.99 N° 13 Abrégé n° 101.708 m) et sont préparés par des méthodes classiques; ils sont préconisés comme traceurs biologiques . L'anhydride dans lequel $A_1$ = $A_2$ = H est décrit dans Venkataraman, K. "The Chemistry of Synthetic Dyes", Academic Press, New York, 1952, Vol.2 . On préfère les composés avec $A_1$ = $A_2$ = $SO_3M$, qui sont très hydrosolubles.

Les haptènes, antigènes utilisés pour préparer les composés fluorescents de formule I, sont des composés susceptibles de donner naissance à des anticorps monoclonaux ou polyclonaux,seuls ou après un couplage à une molécule immunostimulante. Leurs masses moléculaires sont généralement comprises entre 100 et 20 000 de telle sorte que les composés de formule I soient solubles en milieu aqueux,et que les mouvements propres de ces molécules en solution soient suffisants pour qu'une lumière à polarisation linéaire donne naissance à une lumière de fluorescence peu polarisée.Tous les haptènes ou antigènes porteurs d'une fonction chimiquement active et répondant aux conditions précédemment citées peuvent être utilisés pour préparer des composés fluorescents selon l'invention, ce qui permet de doser de nombreuses molécules . Le procédé de l'invention est particulièrement adapté au dosage des produits présents en faible concentration dans les milieux biologiques,tels que sang, urine, liquide céphalorachidien , ou dans les liquides obtenus après un traitement physico-chimique de ces milieux et pour lesquels les procédés de dosage simples, précis et peu coûteux sont rares, alors que l'importance clinique des produits est manifeste. Parmi eux, on citera les stéroïdes naturels ou synthétiques ,les neuro-médiateurs,les hormones polypeptidiques comme l'insuline,les prostaglandines ,les allergènes, les toxines bactériennes,mais aussi des médicaments dont il est important de suivre la cinétique de répartition et le métabolisme dans l'organisme,tels que les cardiotropes,les antibiotiques.

Certains haptènes doivent être couplés à des protéines pour déclencher l'apparition d'anticorps in vivo et ce couplage se fait par l'intermédiaire de groupes réactifs classiques dans le domaine de l'immunologie , comme $-C=N-O-CH_2-COOH$ à partir des carbonyles ou $-O-CO-(CH_2)_2-COOH$ à partir des hydroxyles des stéroïdes.L'invention a aussi pour objet de tels composés. Dans ce cas, on préfère que le composé fluorescent de formule I soit préparé avec le groupe haptène ainsi modifié , c'est-à-dire porteur du groupe utilisé pour le couplage au composé immunostimulant, et en outre que la fixation de cet haptène modifié sur le noyau fluorescent se fasse par l'intermédiaire de ce groupe modificateur réactif . On a en effet constaté que les anticorps reconnaissent mieux les haptènes couplés aux composés fluorescents par une fonction homologue, que par une fonction hétérologue.

Dans le cas où l'haptène à doser ne comporte pas une fonction pouvant réagir sur les composés fluorescents de départ II ou III, on greffera sur l'haptène , préalablement à la préparation,un groupe réactif tel qu'un aminoacide sur une fonction cétone d'un haptène.

Dans le cas où le composé à doser a une masse moléculaire supérieure à 60 000 environ,on ne fixera pas sur le composé fluorescent de départ le composé à doser lui-même mais un fragment purifié d'un anticorps monoclonal dirigé contre ce composé et reconnaissant un épitope de l'antigène à doser. Parmi ces fragments on peut citer les fragments Fab; tous ces fragments comportent des fonctions amines primaires et pourront être fixés sur le composé fluorescent de départ par les moyens décrits ci-dessus. Par ce moyen, on pourra doser des antigènes ayant des masses moléculaires supérieures à 60 000, comme l'albumine, les immunoglobulines A, G, M et E, certaines enzymes, l'alpha-foetoprotéine, l'antigène carcinoembryonaire ou des virus, et bactéries.

Le fragment d'anticorps devra provenir d'un anticorps monoclonal ayant une affinité suffisante pour l'antigène à doser, pour permettre un dosage dont la sensibilité soit adaptée à la concentration des antigènes dans les liquides biologiques.

Les composés fluorescents de formule I sont préparés par des méthodes connues en elles-mêmes; on peut faire réagir les deux produits : haptène (ou un de ses dérivés), antigène ou fragment d'anticorps et composé de formule II ou de formule III, en quantités stoechiométriques, mais on préfère pour faciliter l'isolement du produit final, mettre un excès du réactif le moins coûteux et/ou le plus facile à déceler. En général, le réactif fluorescent I est purifié par chromatographie d'adsorption solide-liquide, en couche mince, en colonne, à haute performance ou non. Les réactions ont lieu en solution, dans des solvants tels qu'alcools, cétones, solvants aprotiques polaires (comme la diméthylformamide)ou l'eau à une température comprise entre 10° C et 50° C environ, en quelques heures; les conditions opératoires, dont le pH du milieu réactionnel, sont évidemment fonction des réactifs en présence.

Un autre objet de l'invention est le composé fluorescent applicable aux dosages immunologiques en polarisation de fluorescence, qui est un dérivé d'(amino-4) naphtalimide de formule I:

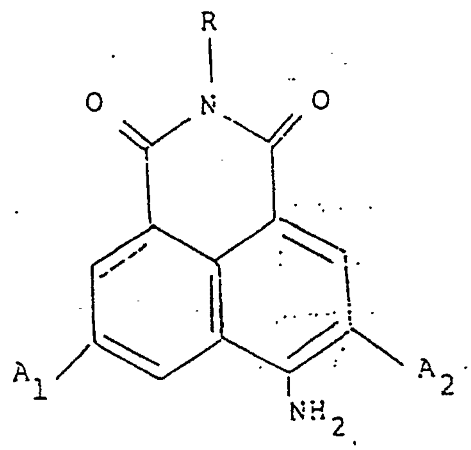

I

dans laquelle :

-$A_1$ à $A_2$, identiques ou différents, représentent l'atome d'hydrogène ou un groupe $SO_3^-$ $M^+$ dans lequel $M^+$ représente un cation alcalin ou ammonium, et -R représente avec l'atome d'azote auquel il est lié, un groupe dérivé d'un haptène, d'un antigène de masse moléculaire inférieure à 20 000 ou d'un fragment d'anticorps et peut résulter de la réaction de l'haptène, l'antigène ou la fraction d'anticorps, avec un composé de formule II :

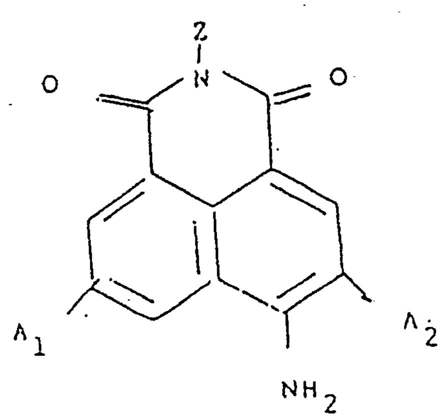

dans laquelle $A_1$ et $A_2$ ont les significations données par le composé de formule I et Z est un groupe susceptible de réagir avec l'haptène, l'antigène ou le fragment d'anticorps sans modification des propriétés immunologiques dudit haptène , antigène ou fragment d'anticorps, ledit réactif étant tel que l'antigène ou la fraction d'anticorps est fixé sur l'azote de l'imide ou sur Z indirectement et l'haptène est fixé sur Z indirectement , c'est-à-dire par l'intermédiaire d'un groupe bifonctionnel , caractérisé en ce que le groupe bifonctionnel est choisi parmi les diamines aliphatiques, l'acide gamma-amino-butyrique ou la cystéamine.

L'invention décrit également le procédé de dosage par immunopolarisation de fluorescence qui met en oeuvre un composé fluorescent de formule I.

Dans le cas où le produit à doser est un haptène ou un antigène de faible masse moléculaire, le procédé est de type par compétition. Il consiste :

1) à introduire dans un volume donné de ce milieu un composé de formule I reconnu par le même anticorps que le produit à doser, en une quantité connue de l'ordre de celle du produit à doser présent dans le milieu, qui a été, si nécessaire, préalablement dilué avec une solution tampon habituellement utilisée en biologie, tel qu'un tampon phosphate à pH 7,

2) à introduire ensuite une quantité donnée d'anticorps dirigés contre le produit à doser et le composé de formule I, quantité inférieure à celle qui serait nécessaire pour complexer tous les antigènes présents,

3) à laisser incuber l'ensemble quelques minutes à une température comprise entre 4°C et 20°C environ, puis

4) à mesurer le taux P de polarisation de fluorescence de l'échantillon ainsi traité, et à déduire de P la

concentration de l'haptène ou antigène étudié dans la solution, à l'aide de courbes d'étalonnage préalablement tracées.

La mesure de P est réalisée avec tout appareil commercial adapté.

Dans le cas des composés fluorescents dérivés des composés de formule II dans laquelle Z est $Z_1$ ou $Z_2$, la longueur d'onde de la lumière incidente est de 425 nm environ, alors que celle de la lumière de fluorescence émise est 540 nm environ.

La concentration mesurable des haptènes ou antigènes dans le milieu lors du dosage, milieu préalablement dilué si nécessaire, dépend de l'appareil de mesure de la polarisation qui est utilisé mais est généralement compris entre 0,2 $\mu$g/l et 10 $\mu$g/l.

On peut doser les Ag présents dans les milieux naturels comme le sérum, le plasma, la salive, le liquide amniotique, le lait, l'urine, sans traitement préalable de ceux-ci, ce qui est particulièrement avantageux ou dans tout autre liquide comme, par exemple,les solutions obtenues après séparation physico-chimique de certains médicaments de leurs métabolites sanguins prélevés in vivo ou issus de cultures cellulaires.

Dans le cas où le produit à doser est un antigène de poids moléculaire élevé et qu'on utilise un composé fluorescent à base d'un fragment Fab de l'un de ses anticorps monoclonaux, le procédé de dosage est de type direct. Il consiste :

1) à introduire dans le milieu à étudier, préalablement dilué si nécessaire, une quantité connue d'un composé fluorescent de formule I porteur du fragment Fab d'un anticorps monoclonal dirigé contre l'antigène à doser,

2) à incuber quelques minutes à une température comprise entre 4° C et 25° C environ,

3) à mesurer le taux P de polarisation de fluorescence du milieu ainsi traité et à déduire de P la concentration de l'antigène présent dans le milieu par comparaison avec des courbes d'étalonnage préalablement tracées.

On a comparé les composés fluorescents de formule I selon l'invention, à d'autres composés fluorescents déjà utilisés pour des dosages en immunopolarisation de fluorescence. Un certain nombre de propriétés ont été étudiées et les résultats sont regroupés dans le tableau I :

- la solubilité aqueuse est un critère important puisque la formation du complexe Ag-Ac doit avoir lieu en milieu aqueux; en milieu organique Ac est dénaturé et perd son affinité pour Ag,
- l'évolution du spectre d'émission ou d'absorption avec le milieu (pH), la nature de l'haptène à doser et l'environnement du fluorophore (polarité du site de liaison, solvants, Ac...) doit être limitée, pour éviter la multiplication des étalonnages,
- la stabilité dans le temps de la molécule du composé fluorescent est indispensable pour son utilisation en coffrets réactifs,
- la différence ($\lambda_{EX}$ - $\lambda_{EM}$) entre les longueurs d'onde d'excitation et d'émission de fluorescence doit être suffisante pour que la méthode soit sensible; en effet lorsque cette différence s'accroit, il est plus facile de supprimer le bruit de fond, résultant de la diffusion de la lumière d'excitation,
- enfin, l'absence d'absorption des rayonnements d'excitation ou d'émission par les milieux où se trouvent les produits à doser est fondamental.

Dans ce qui suit, on décrit les exemples de composés fluorescents selon l'invention et leur application au dosage de l'haptène ou de l'antigène correspondant.

Le dosage par compétition a été étudié en traçant les courbes de saturation et de déplacement

**TABLEAU I**

| critère / composé fluorescent préparé avec | Solubilité aqueuse | Evolution du spectre | Stabilité | $\lambda_{EX}-\lambda_{EM}$ (nm) | Absorption milieu | Référence de l'étude |
|---|---|---|---|---|---|---|
| Fluoresceïne et dérivés | + (moyenne) | avec pH | faible | 495-525 (trop faible) | non | -J. Immunol. 93, p. 232 (1964) -FR 2 500 165 |
| Dansyl | + | avec environnement | bonne | 340-520 | oui | Arch. Biochem.Biophys. 128 p. 163 (1968) |
| Iodoaminonitrobenzodiazole | faible | avec haptène environnement pH | faible | 495-530 (trop faible) | non | Biophys. J. 32, 223 (1980) |
| Chlorure de nitrobenzodiazole | + | id | sensible à la lumière | 470-530 | non | J. Mol. Biol. 97, 423 (1975) |
| Acide nitrobenzodiazole aminohexanoïque | + | avec haptène et environnement | faible | 470-530 | non | Life Sciences 21, 345 (1977) |
| Acide OH-7-coumarine-3 carboxylique | faible | avec pH et environnement | faible | 333-445 325-396 | oui | J. Steroid Biochem. 19, p. 1811 (1983) |
| (Amino-4 disulfonato-3,6) naphtalimide | + + (bonne) | nulle | bonne | 425-540 (important) | non | |

avec divers haptènes et composés fluorescents étudiés, tandis que le dosage direct des Ag de forte masse moléculaire a été étudié en traçant les courbes de saturation avec l'antigène à doser.

Dans le premier cas,la courbe de saturation représente l'évolution de la polarisation P d'une solution d'un composé fluorescent en tampon phosphate aqueux, dans laquelle on ajoute des quantités connues d'anticorps dirigés contre l'haptène, ou l'antigène, désignés par Ag, ayant servi à la préparation du

composé fluorescent. Cette courbe sensiblement sigmoïdale traduit l'existence de la liaison immunogénique entre composé fluorescent et anticorps; la différence entre P lue entre l'absence d'Ac et P max en présence d'Ac doit être d'au moins 0,05 unité pour que le composé fluorescent étudié et l'anticorps correspondant soient utilisables dans un procédé de dosage par immunopolarisation de fluorescence.

La courbe de déplacement représente l'évolution du P d'une solution d'un mélange d'un composé fluorescent de formule I avec une quantité donnée d'anticorps correspondants Ac, lorsque l'on ajoute des quantités connues du Ag correspondant. La quantité de Ac est choisie de préférence de telle sorte que P de la solution du composé I + Ac soit environ les 2/3 de P max déterminé sur la courbe de saturation; la quantité d'Ag ajoutée est de préférence comprise entre 0,1 et 10 fois environ, en moles, la quantité de composé fluorescent.

Une telle courbe montre qu'il y a compétition entre Ag et le composé fluorescent correspondant pour la fixation sur leur anticorps; elle traduit la sensibilité du procédé de dosage utilisant ces 3 réactifs; c'est aussi la courbe étalon qui est utilisée lors du dosage de l'Ag dans un milieu liquide quelconque, pour déduire la concentration d'Ag présent dans l'échantillon à étudier, de la valeur de la polarisation mesurée.

Dans le cas du dosage direct, la courbe de saturation représente l'évolution de P pour une solution contenant une quantité donnée du composé fluorescent I, comportant un fragment d'anticorps, à laquelle on ajoute des quantités connues de l'antigène, reconnu par le fragment d'anticorps. Cette courbe traduit l'existence d'une liaison immunogénique entre le composé fluorescent et l'antigène. La différence de P entre deux solutions dont l'une ne contient pas d'Ag et l'autre est saturée d'Ag, doit être d'au moins 0,01 unité pour que le composé fluorescent soit utilisable dans un procédé de dosage par immunopolarisation de fluorescence compte tenu de la sensibilité des appareils connus.

Exemple 1.

Dans cet exemple, l'haptène est la testostérone.

a) Préparation au composé fluorescent.

On dissout 1,03 mg (3,6 µmoles) de testostérone dans 500 µl de méthanol, contenant 10 % (V/V) d'acide acétique, et 3,9 mg (8,5 µmoles) d'amino-4 N-(hydrazinocarbonyl amino) naphtalimide-3,6 disulfonate de lithium (composé de formule II avec

$$Z : NH-\underset{\underset{O}{\|}}{C}-NH-NH_2 \ ;$$

$A_1 = A_2 = SO_3^{\ominus}M^{\oplus}$ et $M^+ = Li^{\oplus}$)

dans 1500 µl du même solvant méthanol/acide acétique. On mélange les deux solutions et abandonne 12 heures à température ambiante avant de porter 6 heures à 37° C. Après quelques heures à température ambiante, on neutralise environ 95 % de l'acide acétique présent par addition de 3,32 ml d'une solution aqueuse N de NaOH. Le suivi de la réaction est facilité lorsque des traces de testostérone marquée au tritium $^3H$ sont introduites au départ. Le mélange est soumis à une chromatographie préparative en couches minces sur plaques de gel de silice (60-Merck®) en éluant avec un mélange chloroforme/méthanol/$H_2O$ (50/49/1 - V/V/V/). Le rf du produit final est 0,49.

Le produit résultant est soumis ensuite à une chromotographie liquide haute performance (HPLC), en phase reverse, sur une colonne de Lichros® 10 RP 18 commercialisé par CHROMPACK (USA) de longueur 250 mm et diamètre 10 mm; en éluant avec une solution aqueuse contenant un tampon phosphate (pH = 8, c = 0,05 M). L'élution est suivie avec un détecteur UV.

b) Etude du composé fluorescent.

1) Courbe de saturation.

On mesure la polarisation P d'une solution du composé préparé selon a) (c = 5,8 $10^{-8}$M) dans un tampon phosphate; puis on ajoute à cette solution des aliquotes d'une solution aqueuse contenant 0,5 mg/ml d'anticorps antitestostérone IgG, et mesure à chaque fois l'augmentation de la polarisation. Les échantillons étudiés ont un volume de 1 ml environ.

Les volumes de solution d'anticorps ajoutés sont choisis de telle sorte que les concentrations finales d'Ac soient comprises entre 3 et 30 µg/ml. La courbe obtenue est l'objet de la Fig. 1.

2) Courbe de déplacement.

On introduit dans des tubes contenant des concentrations de testostérones, comprises entre 0,67 et 33,3 ng/ml, une quantité constante de composé fluorescent préparé selon a) et 17,5 µg d'IgG par tube, de telle sorte que le volume final de solution dans les tubes soit 1 ml et la concentration finale de composé fluorescent $10^{-8}$ M. On laisse incuber 10 minutes à 4° C avant de mesurer P.

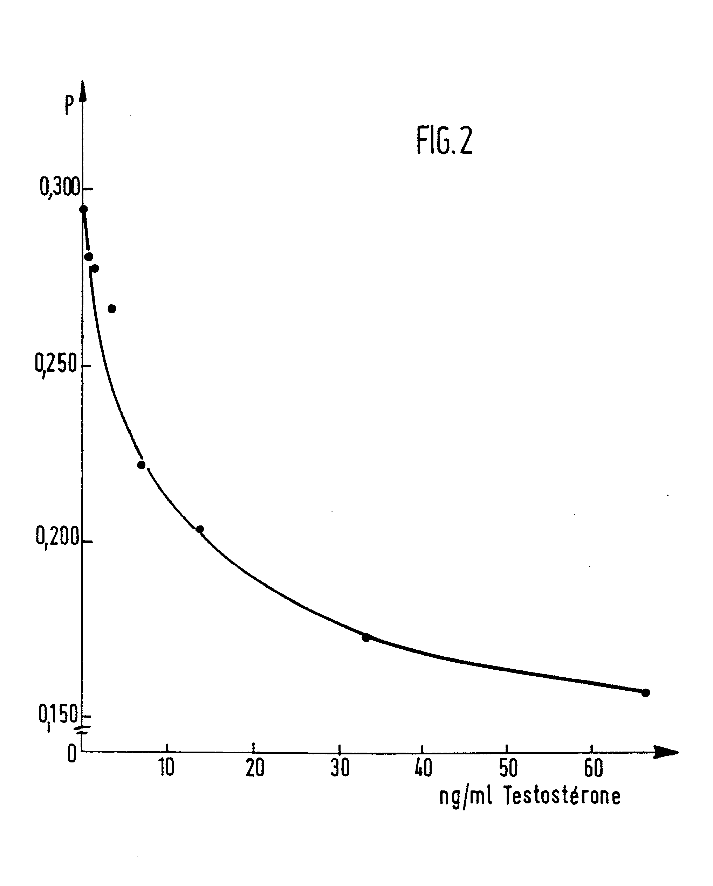

La courbe de décroissance de P en fonction de la quantité d'haptène est représentée fig. 2.

Exemple 2.

Dans cet exempte l'haptène a doser est la testostérone et le produit utilisé pour préparer le composé fluorescent de formule I est la testostérone sur laquelle on a greffé l'acide aminooxyacétique par réaction sur le carbonyle en 3 en formant un groupe = $NOCH_2$-COOH; la testostérone ainsi modifiée est appelée testostérone-3 CMO. Ce greffage permet le couplage de l'hormone à une protéïne et la préparation d'un anticorps antitestostérone suivant une technique habituelle, décrite notamment par ERLANGER et coll. dans J. Bio Chem. 228 p 713 (1957).

a) Préparation du composé fluorescent.

On dissout 1 mg (2,8 µmole) de testostérone-3 CMO dans 60 µl de dioxanne et ajoute 20 µl d'une solution préparée à partir de 6,6 µl de tri-n-butylamine et 200 µl de dioxanne. Après 10 minutes d'agitation à 4° C, on ajoute 20 µl d'une solution de chlorocarbonate d'isobutyle, préparée avec 3,8 µl de chlorocarbonate d'isobutyle et 200 µl de dioxanne. Après 30 minutes d'agitation à 10° C, l'anhydride mixte ainsi préparé est mis à réagir avec le composé de formule II dans lequel $Z = Z_1$, $A_1 = A_2 = SO_3^{\ominus}M^{\oplus}$ et $M^{\oplus} = Li^{\oplus}$.Pour cela on introduit 50 µl de la solution d'anhydride mixte dans 250 µl d'une solution de 0,76 mg du composé II dans un tampon aqueux carbonate-bicarbonate 0,1 M (pH = 8,5) puis lentement 200 µl de dioxanne.

Après 10 heures environ sous agitation à 10° C, le composé fluorescent est isolé par une chromatographie en phase liquide à haute performance sur support Lichros®10 RP 18, avec une colonne de longueur 250 mm et de diamètre 10 mm, et en utilisant comme éluant un mélange de tampon phosphate 0,05 M (pH = 8) et méthanol dont la composition a été modifiée au cours de ta chromatographie comme suit :

| | | |
|---|---|---|
| temps = 0 | 0 % $CH_3OH$ | (tampon seul) |
| t = 10 min | 45 % $CH_3OH$ | (V/V) |
| t = 25 min | 55 % $CH_3OH$ | |
| t = 40 min | 55 % $CH_3OH$ | |
| t = 45 min | 65 % $CH_3OH$ | |

On suit l'évolution de la chromatographie par étude de l'absorption d'un rayonnement de longueur d'onde 430 nm.

b) Etude du composé fluorescent obtenu.

- Les valeurs obtenues lors du tracé des courbes de saturation et de déplacement pour une concentration de 5,8 x $10^{-8}$ M du composé fluorescent dans la solution et une concentration d'anticorps dans le second cas de 0,03 mg/ml figurent dans le tableau II ci-après :

TABLEAU II

| Courbe de saturation | | Courbe de déplacement | |
|---|---|---|---|
| Ac mg/ml | P | Ag ng/ml | P |
| 0 | 0,028 | 0 | 0,235 |
| 0,0033 | 0,049 | 0,67 | 0,227 |
| 0,0067 | 0,082 | 1,33 | 0,208 |
| 0,0100 | 0,123 | 3,33 | 0,194 |
| 0,0133 | 0,165 | 6,7 | 0,170 |
| 0,0167 | 0,202 | 13,3 | 0,136 |
| 0,0200 | 0,249 | 33,3 | 0,101 |
| 0,0233 | 0,282 | | |
| 0,0267 | 0,302 | | |
| 0,0333 | 0,322 | | |

Exemple 3.

Dans cet exemple, l'haptène à doser est la testostérone; le composé fluorescent est préparé avec la testostérone-3 CMP qui est par ailleurs utilisée pour préparer des anticorps après couplage à la sérum albumine bovine selon une méthode connue, et avec le composé de formule II dans lequel Z est $Z_2$, ces deux produits étant couplés par réaction d'une molécule bifonctionnelle le diamino-1,7 heptane.

a) Préparation du composé fluorescent.

- 1ère méthode : on introduit dans une solution de 108 mg de testostérone-3 CMO dans 3 ml de dioxanne, 71 µl de tri-n-butylamine. Après 10 minutes d'agitation à 4° C, on ajoute 40 µl de chlorocarbonate d'isobutyle pur et l'on maintient sous agitation 30 minutes à 10° C, avant de verser lentement cette solution en 2 heures environ à T = 10° C, dans une solution de diamino-1,7 heptane préparée avec 547 mg de diamine, 22 ml d'eau et 18 ml de dioxanne.

  Après 12 heures d'agitation à 10° C, on extrait par l'éther éthylique, la testostérone, portant en position 3 à la place du carbonyle, le groupe $NOCH_2CONH(CH_2)_7NH_2$, et on purifie ce composé par chromatographie liquide haute performance (HPLC). On fait alors réagir cette amine sur le composé de formule II dans lequel Z = $Z_2$ (vinylsulfone)$A_1 = A_2 = SO_3^{\ominus}$ $M^{\oplus}$ et $M^{\oplus} = Li^{\oplus}$. Pour cela, on dissout 2 mg du composé II dans 1 ml d'eau, et introduit cette solution dans un tube contenant 1,31 mg du dérivé de testostérone en solution dans 1 ml de tampon phosphate 0,1 M. On ajuste ensuite le volume à 4 ml par une solution de bicarbonate de sodium 0,2 M. Après plusieurs heures à température ambiante un précipité se forme. On introduit alors 2 ml d'éthanol dans le milieu pour solubiliser l'ensemble et on abandonne 4 heures. Le composé fluorescent final est alors purifié par HPLC comme précédemment.
- 2ème méthode : On prépare une solution de diamino-1,7 heptane par introduction de 80 mg de l'amine dans 18,6 ml d'une solution aqueuse 0,1 M de $NaH_2PO_4$; le pH final est de 7.

  Par ailleurs, on dissout 10 mg du composé II avec Z = $Z_2$ dans 5 ml d'eau; cette solution est introduite lentement dans 7 ml de la solution de diamine auxquels ont été préalablement ajoutés 12 ml de tampon bicarbonate de sodium; après 2 heures d'agitation à 20° C, le pH s'est élevé à 8,4 et on introduit une solution aqueuse de $NaH_2PO_4$ pour le redescendre à 8. Après 1 heure, il se forme un précipité, qui est éliminé par centrifugation et le composé de formule II dans laquelle :

Z :
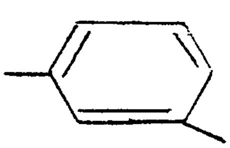
$SO_2 - CH_2 - CH_2NH - (CH_2)_7-NH_2$

contenu dans le surnageant est purifié par HPLC.

On fait ensuite réagir 1,88 mg de ce composé en solution dans un tampon bicarbonate de sodium 0,1 M de pH = 8,2 avec 1 mg d'un anhydride mixte dérivé de la testostérone-3 CMO.

b) Etude du composé fluorescent obtenu.

On a effectué les mesures de polarisation pour des solutions contenant 0,9 x $10^{-7}$M de composé fluorescent, avec pour l'expérience conduisant au tracé de la courbe de déplacement une concentration en anticorps de 0,015 mg/ml.

Les résultats obtenus figurent dans le tableau III suivant :

TABLEAU III

| Courbe de saturation | | Courbe de déplacement | |
|---|---|---|---|
| Ac mg/ml | P | Ag ng/ml | P |
| 0 | 0,0365 | 0 | 0,226 |
| 0,0033 | 0,0548 | 1,33 | 0,203 |
| 0,0067 | 0,0632 | 3,33 | 0,222 |
| 0,0100 | 0,0826 | 6,70 | 0,160 |
| 0,0133 | 0,127 | 13,30 | 0,108 |
| 0,0167 | 0,187 | 33,30 | 0,073 |
| 0,0200 | 0,211 | 66,70 | 0,066 |
| 0,0233 | 0,219 | | |
| 0,0267 | 0,243 | | |
| 0,0300 | 0,220 | | |
| 0,0333 | 0,218 | | |

Exemple 4.

Dans cet exemple l'haptène à doser est l'oestriol. Il est utilisé pour préparer le composé fluorescent sous forme de son dérivé d'oestriol-6 CMO, qui sert par ailleurs à la préparation de l'anticorps, selon des procédés connus.

a) Préparation du composé fluorescent.

On dissout 1 mg (2,58 µmole) d'oestriol-6 CMO dans 100 µl de dioxanne et on ajoute 20 µl d'une solution préparée à partir de 12,1 µl de tri-n-butylamine et de 400 µl de dioxanne. Après 10 min d'agitation à 4° C on ajoute 20 µl d'une solution de chlorocarbonate d'isobutyle préparée avec 3,5 µl de carbonate et 200 µl de dioxanne. Après 30 min d'agitation à 10° C l'anhydride mixte ainsi préparé est mis à réagir avec le composé de formule II dans lequel $Z = Z_1$ $A_1 = A_2 = SO_3^{\ominus}M^{\oplus}$ et $M^{\oplus} = Li^{\oplus}$. Pour cela on introduit la solution d'anhydride mixte dans 420 µl d'une solution de 1,2 mg du composé II dans un tampon aqueux carbonate bicarbonate 0,1 M (pH = 8,5).

Après 10 h environ sous agitation à 10° C, le composé fluorescent est isolé (Rf 0,65) sur une plaque de chromatographie en couche mince (gel de silice 60 Merck) en éluant avec dichlorométhane/méthanol (50/50).

b) Etude du composé fluorescent obtenu.

On a étudié la polarisation de fluorescence pour des solutions contenant $9.10^{-8}$ M du précédent composé fluorescent; pour l'expérience conduisant au tracé de la courbe de déplacement, la concentration en anticorps correspondant était de 12 µg/ml. Les résultats obtenus figurent dans le tableau IV.

TABLEAU IV

| Courbe de saturation | | Courbe de déplacement | |
|---|---|---|---|
| Ac mg/ml | P | Ag ng/ml | P |
| 0 | 0,0183 | 0 | 0,134 |
| 0,0027 | 0,0190 | 0,67 | 0,128 |
| 0,0053 | 0,0249 | 1,33 | 0,134 |
| 0,0080 | 0,0493 | 3,33 | 0,125 |
| 0,0107 | 0,0852 | 6,70 | 0,120 |
| 0,0133 | 0,1160 | 13,30 | 0,097 |
| 0,0160 | 0,1290 | 33,30 | 0,078 |
| 0,0187 | 0,132 | 66,70 | 0,075 |
| 0,0213 | 0,135 | | |

Exemple 5.

Dans cet exemple,l'haptène à doser est la sérotonine; on utilisera le procédé par compétition. Pour cela, le composé fluorescent peut être préparé comme suit :

on dissout 1 mg (4,5 µmoles) de sérotonine dans 1 ml d'eau distillée et 2,48 mg (4,5 µmoles) du composé II ($Z = Z_2$) dans 1,25 ml d'eau distillée. Les deux solutions sont mélangées et on ajuste ensuite le volume à 4 ml par une solution aqueuse de bicarbonate de sodium (0,2 M).

Après 16 heures sous agitation à l'abri de la lumière et à la température ambiante, le composé fluorescent (Rf = 0,70) est isolé sur une plaque préparative de chromatographie en couche mince (PLK 5-Gel de Silice-80 Å-WHATMAN®) en éluant avec propanol/eau 64/36. La sérotonine liée au composé II est mise en évidence par vaporisation d'une solution éthanolique de l'aldéhyde cinnamique, réactif spécifique du noyau indole.

Exemple 6.

Dans cet exemple, l'antigène à doser est l'insuline, par le procédé par compétition. Le composé fluorescent I est préparé comme suit : on dissout 2,3 mg (0,38 µmole ) d'insuline de porc dans 0,5 ml de bicarbonate (0,2 M) et 1 mg (1,8 µmole) du composé II ($Z = Z_2$) dans 0,5 ml du tampon phosphate pH 7 (0,05 M). Les deux solutions sont mélangées et on rajoute 1 ml de bicarbonate de sodium (0,2 M).

Le mélange est abandonné une heure à la température ambiante sous agitation douce, 12 heures à 4° C et une heure à la température ambiante.

Le composé fluorescent est isolé par une chromatographie sur colonne de BIO-GEL $P_2$ (BIO-RAD®) en éluant avec du tampon phosphate pH 7 (0,05 M). Le composé fluorescent passe dans le volume d'exclusion.

Exemple 7.

Dans cet exemple, la molécule à doser est la choriomammotropine humaine (hCS) ou hormone placentaire lactogène (hPL).

a) Préparation du composé fluorescent

L'hormone placentaire lactogène provenant de chez ICN Pharmaceuticals (CLEVELAND, OHIO) est mise en solution dans un tampon carbonate-bicarbonate (0,1 M) pH 9,0 à la concentration de 10 mg/ml : 11 mg d'hPL (0,509 µmole ) dans 1,1 ml de tampon.

Par ailleurs, on dissout 3 mg de composé de formule II avec Z = $Z_2$ (5,45 µmoles) dans 1 ml de tampon carbonate-bicarbonate (0,1 M)pH 9,0, et on le conserve à l'abri de la lumière.

Deux taux de marquage sont obtenus comme suit en mettant en présence les deux solutions précédentes en proportions différentes, sous agitation douce pendant 3 heures à 18° C puis 18 à 20 heures à 5° C environ :

- Composé 1 : à 0,45 ml de la solution d'hPL à 10 mg/ml, on ajoute 0,2 ml de la solution du composé fluorescent à 3 mg/ml et on ajuste le volume final à 2 ml avec le tampon carbonate-bicarbonate, 0,1 M, pH 9,0.Le rapport molaire des deux composés mis en présence est de 4,8 moles de composé fluorescent de départ par mole d'hormone.
- Composé 2 : à 0,45 ml de la solution d'hPL à 10 mg/ml, on ajoute 0,4 ml de la solution du composé fluorescent à 3 mg/ml et on ajuste le volume final à 2 ml avec le tampon carbonate-bicarbonate 0,1 M pH 9,0; soit un rapport molaire des deux composés mis en présence de 9,5 moles de composé fluorescent de départ par mole d'hormone.

Elimination du composé fluorescent de départ n'ayant pas réagi.

Les deux préparations (couplage 1 et 2) sont soumises à une filtration sur gel par passage sur une colonne de 1,5 x 12 cm de Biogel $P_2$ (BIO RAD Lab. RICHMONDS, CA). L'élution est faite avec un tampon phosphate, 0,05 M, pH 7,0 à un débit de 20 ml/heure. Le composé fluorescent de départ est exclu et sort entre le 9ème et le 16ème ml, le colorant est élué entre les 167-224ème ml.

b) Etude du composé fluorescent.

On détermine, pour chacun des deux composés fluorescents obtenus, le degré de marquage (quantité de colorant par molécule à doser); la quantité de protéine étant dosée par la méthode de Lowry, O. H., Rosebrough, N.J., Farr, A.L. et Randal, R.J., "J. biol. Chem." 193 (1951) 265 et celle de colorant étant déterminée par mesure de l'absorption du composé obtenu à 428 nm.

Les composés 1 et 2 présentent des taux de marquage de 1,53 et 3,20 respectivement.

Une étude comparative des deux composés a mis en évidence, pour une excitation à 425 nm, une émission de fluorescence maximale vers 535 nm, avec un signal fluorescent maximum pour le composé 2.

1) Courbe de saturation :

On mesure la polarisation P d'une solution du composé 2 telle que la concentration de hPL soit 2,3 $10^{-8}$ M tandis que celle du composé fluorescent de départ soit 7,40 $10^{-8}$ M, dans un tampon phosphate, 0,02 M, pH 7,0.

On introduit ensuite dans cette solution des aliquotes d'une solution aqueuse d'immunoglobulines du lapin anti-hPL à 0,18 mg/ml puis à 1,8 mg/ml de sorte que les concentrations finales en immunoglobulines soient comprises entre 2,2 et 430 µg/ml.

Les valeurs de P obtenues après chaque addition sont rapportées dans le tableau V.

2) Courbe de déplacement.

On introduit dans des tubes contenant chacun une quantité donnée de hPL, les concentrations étant comprises entre 0,05 et 10 µg/ml, une quantité constante du composé fluorescent 2 préparé selon a) et 45 µg/ml d'IgG anti-hPL de telle sorte que le volume final soit de 2 ml et la concentration finale du composé fluorescent de 7,4 $10^{-8}$ M (mesuré par fluorescence). On laisse incuber 2 à 10 heures à 5° C ± 2° C avant de mesurer P.

Les valeurs de P obtenues figurent dans le tableau V.

TABLEAU V

| Courbe de saturation | | Courbe de déplacement | |
|---|---|---|---|
| IgG µg/ml | P | hPL µg/ml | P |
| 0 | 0,260 | 0 | 0,273 |
| 2,2 | 0,264 | 0,1 | 0,272 |
| 4,4 | 0,267 | 0,2 | 0,261 |
| 30 | 0,291 | 0,4 | 0,250 |
| 50 | 0,305 | 0,75 | 0,249 |
| 70 | 0,313 | 1,5 | 0,235 |
| 177 | 0,307 | 2 | 0,230 |
| 263 | 0,312 | 4 | 0,227 |
| 430 | 0,317 | 10 | 0,227 |

Exemple 8.

Dans cet exemple l'antigène à doser est le conjugué aldostérone-sérum albumine bovine; on utilisera le procédé direct. Pour cela, le composé fluorescent peut être préparé comme suit :

On prépare les fragments Fab d'un anticorps monoclonal de souris anti-aldostérone par digestion avec la papaïne, comme décrit par Mage, M.G. dans "Methods in Enzymology" Tome 70, eds. H. van Vunakis et J.J. Langome, Academic Press, New York, 1980, p. 142. Les fragments Fab sont purifiés par gel filtration et par chromatographie d'affinité sur Protéïne-A-Sépharose. A 0,2 ml d'une solution des fragments Fab 0,1 mM (5 mg/ml) dans un tampon carbonate/bicarbonate 0,025 M, pH 9,2 est ajouté 0,02 ml d'une solution de Composé II ($Z=Z_2$) 2 mM (0,54 mg/ml) dans le même tampon. Le mélange est incubé 18 heures à 4° C, puis filtré sur une colonne (volume 10 ml) de Biogel P-2 avec un tampon phosphate 0,02 M, pH 7,0. Par ce procédé, le rapport molaire du Composé II/Fab dans le produit final (Composé II-Fab) est de 1,3/I.

La courbe de titration pour l'immunogène aldostérone-albumine donne les résultats suivants :

COURBE DE LIAISON

| Aldostérone-albumine µg/ml | P |
|---|---|
| 0 | 0,300 |
| 10 | 0,327 |
| 20 | 0,333 |
| 30 | 0,335 |
| 40 | 0,337 |
| 50 | 0,339 |
| 60 | 0,339 |

Exemple 9.

Dans cet exemple l'antigène à doser est la IgM humaine; on utilisera le procédé direct. Pour cela, le composé fluorescent peut être préparé comme suit :

On prépare et purifie les fragments Fab d'un anticorps monoclonal de souris anti-IgM humaine comme dans l'Exemple 8.

A 0,2 ml d'une solution des fragments Fab 0,1 mM (5 mg/ml) dans un tampon carbonate/bicarbonate 0,025 M, pH 9,2 est ajouté 0,03 ml du composé II ($Z = Z_2$) 2 mM (0,54 mg/ml) dans le même tampon. Le mélange est incubé 18 heures à 4° C, puis filtré sur une colonne (volume 10 ml) de Biogel P-2 avec un tampon phosphate 0,02 M, pH 7,0. Par ce procédé, le rapport molaire du Composé II/Fab dans le produit final (Composé II-Fab) est de 2,4/I.

La courbe de titration de l'IgM humaine donne les résultats suivants :

COURBE DE LIAISON

| IgM µg/ml | P |
|---|---|
| 0 | 0,227 |
| 10 | 0,231 |
| 20 | 0,236 |
| 40 | 0,238 |
| 60 | 0,242 |
| 80 | 0,243 |
| 100 | 0,244 |

## Revendications

1. Procédé de dosage immunologique en polarisation de fluorescence, caractérisé en ce que le réactif de dosage utilisé est un dérivé d'(amino-4) naphtalimide de formule I :

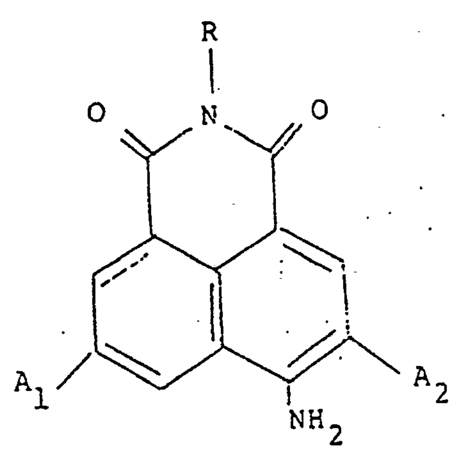

dans laquelle :

- $A_1$ et $A_2$ ,identiques ou différents, représentent l'atome d'hydrogène ou un groupe $SO_3^- M^+$ dans lequel $M^+$ représente un cation alcalin ou ammonium, et

- R représente, avec l'atome d'azote auquel il est lié, un groupe dérivé d'un haptène , d'un antigène de masse moléculaire inférieure à 20 000 ou d'un fragment d'anticorps.

**2.** Procédé selon la revendication 1, caractérisé en ce que dans la formule I, $A_1$ et $A_2$ représentent simultanément $SO_3^- M^+$ , dans lequel $M^+$ représente un cation alcalin ou ammonium.

**3.** Procédé selon la revendication 1, caractérisé en ce que dans la formule I R résulte de la réaction d'un haptène, d'un antigène ou d'une fraction d'anticorps, avec un composé de formule II :

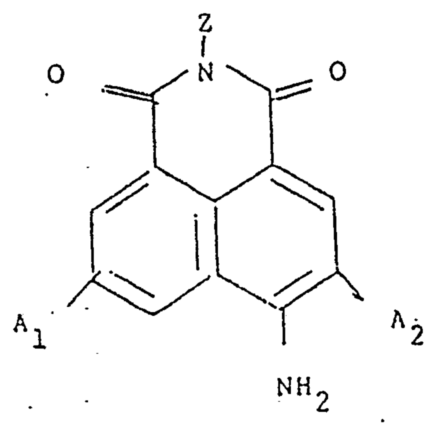

II

dans laquelle $A_1$ et $A_2$ ont les significations données à la revendication 1 et Z est un groupe susceptible de réagir avec l'haptène, l'antigène ou le fragment d'anticorps sans modification des propriétés immunologiques dudit haptène, antigène ou fragment d'anticorps.

**4.** Procédé selon la revendication 3, caractérisé en ce que dans la formule II Z est choisi parmi :

$$-NH-\underset{\underset{O}{\|}}{C}-NH-CH_2$$ ; et

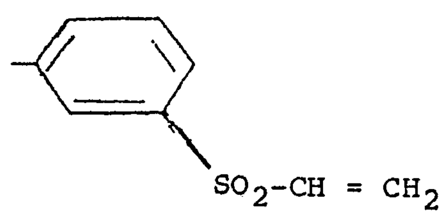

**5.** Procédé selon l'une des revendications 1 à 4 , dans lequel l'haptène, l'antigène ou la fraction d'anticorps est fixé sur l'azote de l'imide ou sur Z indirectement, c'est-à-dire par l'intermédiaire d'un groupe bifonctionnel.

**6.** Procédé selon la revendication 1,caractérisé en ce que le réactif utilisé résulte de l'action d'un haptène , d'un antigène, d'un fragment d'anticorps porteurs d'une fonction amine primaire ou de l'un de leurs dérivés porteur d'une fonction amine primaire, sur l'anhydride de formule III:

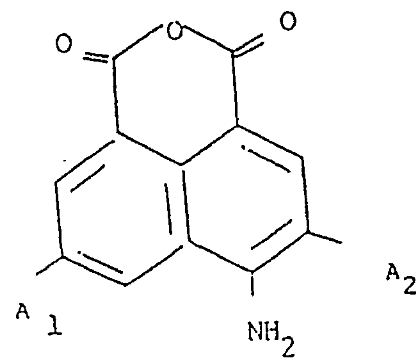

III

dans laquelle $A_1$ et $A_2$ ont les significations données à la revendication 1.

7. Composé fluorescent applicable aux dosages immunologiques en polarisation de fluorescence, selon l'une quelconque des revendications 1 à 6, qui est un dérivé d'(amino-4) naphtalimide de formule I:

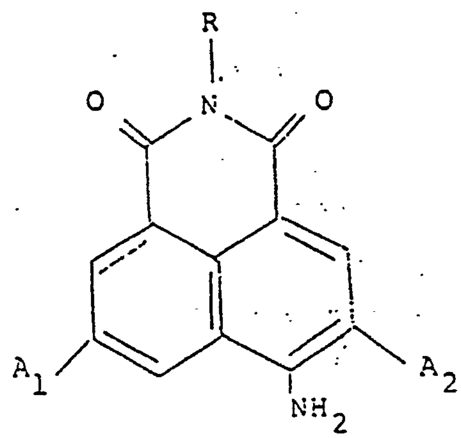

I

dans laquelle :

- $A_1$ à $A_2$, identiques ou différents, représentent l'atome d'hydrogène ou un groupe $SO_3^-$ $M^+$ dans lequel $M^+$ représente un cation alcalin ou ammonium, et
- R représente avec l'atome d'azote auquel il est lié, un groupe dérivé d'un haptène, d'un antigène de masse moléculaire inférieure à 20 000 ou d'un fragment d'anticorps et peut résulter de la réaction de l'haptène, l'antigène ou la fraction d'anticorps, avec un composé de formule II :

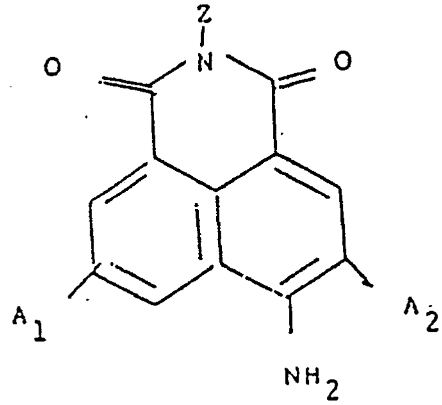

dans laquelle $A_1$ et $A_2$ ont les significations données par le composé de formule I et Z est un groupe susceptible de réagir avec l'haptène, l'antigène ou le fragment d'anticorps sans modification des propriétés immunologiques dudit haptène , antigène ou fragment d'anticorps, ledit réactif étant tel que l'antigène ou la fraction d'anticorps est fixé sur l'azote de l'imide ou sur Z indirectement et l'haptène est fixé sur Z indirectement , c'est-à-dire par l'intermédiaire d'un groupe bifonctionnel , caractérisé en ce que le groupe bifonctionnel est choisi parmi les diamines aliphatiques, l'acide gamma-amino-butyrique ou la cystéamine.

**8.** Composé selon la revendication 7,dans lequel un dérivé de l'haptène, ou de l'antigène à doser, est fixé sur le composé fluorescent de départ .

**9.** Composé selon la revendication 8,dans lequel le dérivé d'haptène est un stéroïde dont un carbonyle a réagi pour donner la fonction $-C=N-O-CH_2COOH$ ou dont un hydroxyle a réagi pour donner la fonction $OCO(CH_2)_2COOH$.

**10.** Procédé de dosage d'haptène ou antigènes de faible masse moléculaire par immunopolarisation de fluorescence, selon l'une quelconque des revendications 1 à 6,caractérisé en ce que:
1)on introduit dans le milieu à étudier une quantité connue d'un réactif de formule I dérivé du produit à doser et reconnu par le même anticorps que le produit à doser,
2)on introduit une quantité donnée d'anticorps dirigés contre le produit à doser et le composé précédemment ajouté,
3)on mesure, après incubation,le taux de polarisation de fluorescence de l'échantillon ainsi traité,
4)on détermine la concentration du produit à doser dans le milieu par référence à des étalons .

**11.** Procédé de dosage d'antigènes de forte masse moléculaire par immunopolarisation de fluorescence selon l'une quelconque des revendications 1 à 6 ,caractérisé en ce que :
1)on introduit dans le milieu à étudier une quantité connue d'un composé fluorescent de formule I comportant un fragment d'un anticorps monoclonal dirigé contre l'antigène à doser, ledit fragment reconnaissant ledit antigène,
2)on incube le mélange,
3)on mesure le taux P de polarisation de fluorescence du milieu ainsi obtenu,
4)on déduit la concentration de l'antigène à doser par référence à des étalons.

## Claims

**1.** Fluorescence polarisation immunoassay method, characterised in that the assay reagent used is a 4-aminonaphthalimide derivative of formula I:

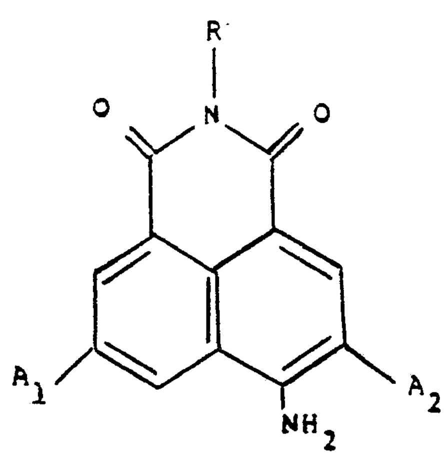

in which:
- $A_1$ and $A_2$, which may be identical or different, represent a hydrogen atom or a group $SO_3^- M^+$ in which $M^+$ represents an alkali metal or ammonium cation, and
- R represents, with the nitrogen atom to which it is attached, a group derived from a hapten, from

an antigen of molecular mass less than 20,000 or from an antibody fragment.

2. Method according to Claim 1, characterised in that, in the formula I, $A_1$ and $A_2$ simultaneously represent $SO_3^- M^+$, in which $M^+$ represents an alkali metal or ammonium cation.

3. Method according to Claim 1, characterised in that, in the formula I, R results from the reaction of a hapten, an antigen or an antibody fragment with a compound of formula II:

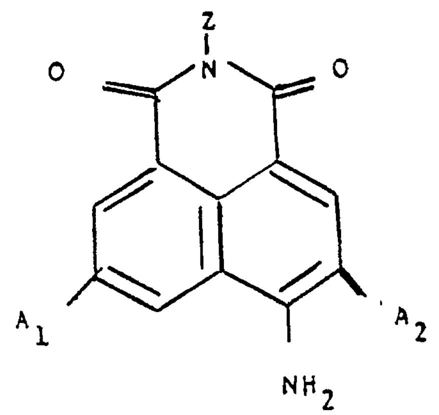

II

in which $A_1$ and $A_2$ have the meanings given in Claim 1 and Z is a group capable of reacting with the hapten, the antigen or the antibody fragment without modification of the immunological properties of the said hapten, antigen or antibody fragment.

4. Method according to Claim 3, characterised in that, in the formula II, Z is selected from:

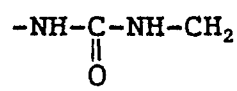

; and

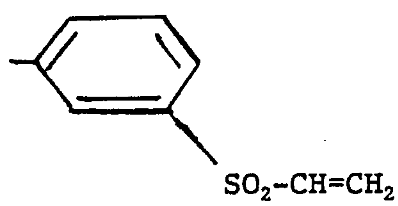

5. Method according to one of Claims 1 to 4, in which the hapten, antigen or antibody fragment is bound to the nitrogen of the imide or to Z indirectly, that is to say via a bifunctional group.

6. Method according to Claim 1, characterised in that the reagent used results from the action of a hapten, an antigen or an antibody fragment bearing a primary amine function, or one of their derivatives bearing a primary amine function, on the anhydride of formula III:

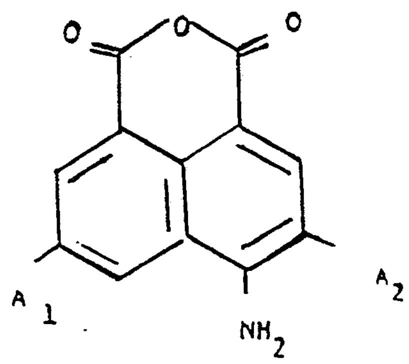

III

in which $A_1$ and $A_2$ have the meanings given in Claim 1.

7. Fluorescent compound applicable to fluorescence polarisation immunoassays, according to any one of Claims 1 to 6, which is a 4-aminonaphthalimide derivative of formula I:

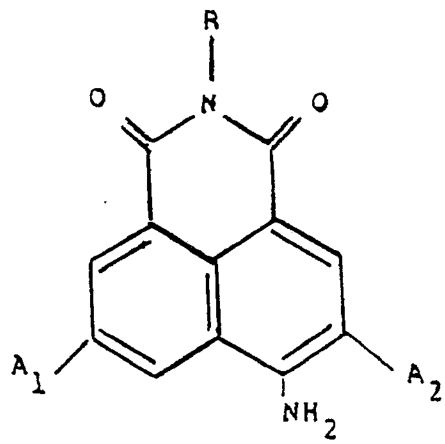

I

in which:

- $A_1$ and $A_2$, which may be identical or different, represent a hydrogen atom or a group $SO_3^- M^+$ in which $M^+$ represents an alkali metal or ammonium cation, and
- R represents, with the nitrogen atom to which it is attached, a group derived from a hapten, from an antigen of molecular mass less than 20,000 or from an antibody fragment, and can result from the reaction of the hapten, antigen or antibody fragment with a compound of formula II:

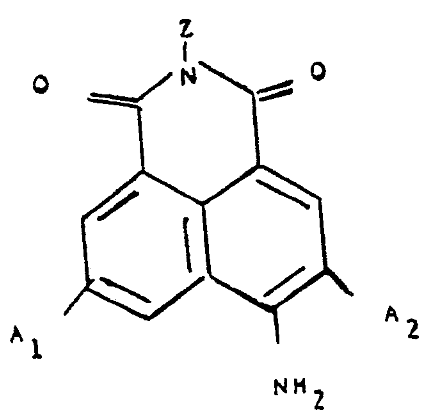

in which $A_1$ and $A_2$ have the meanings given by the compound of formula I and Z is a group capable of

reacting with the hapten, antigen or antibody fragment without modification of the immunological properties of the said hapten, antigen or antibody fragment, the said reagent being such that the antigen or antibody fragment is bound to the nitrogen of the imide or to Z indirectly and the hapten is bound to Z indirectly, that is to say via a bifunctional group, characterised in that the bifunctional group is selected from aliphatic diamines, gamma-aminobutyric acid and cysteamine.

**8.** Compound according to Claim 7, in which a derivative of the hapten or of the antigen to be assayed is bound to the starting fluorescent compound.

**9.** Compound according to Claim 8, in which the hapten derivative is a steroid, a carbonyl of which has reacted to give the function $-C=N-O-CH_2COOH$ or a hydroxyl of which has reacted to give the function $OCO(CH_2)_2COOH$.

**10.** Method for the assay of a hapten or antigen of low molecular mass by fluorescence immunopolarisation according to any one of Claims 1 to 6, characterised in that:

1) a known quantity of a reagent of formula I, derived from the product to be assayed and recognised by the same antibody as the product to be assayed, is introduced into the medium under study,

2) a given quantity of antibodies directed towards the product to be assayed and the compound previously added is introduced,

3) after incubation, the degree of fluorescence polarisation of the sample thus treated is measured,

4) the concentration of the product to be assayed in the medium is determined by reference to standards.

**11.** Method for the assay of antigens of high molecular mass by fluorescence immunopolarisation according to any one of Claims 1 to 6, characterised in that:

1) a known quantity of a fluorescent compound of formula I containing a fragment of a monoclonal antibody directed towards the antigen to be assayed, the said fragment recognising the said antigen, is introduced into the medium under study,

2) the mixture is incubated,

3) the degree P of fluorescence polarisation of the medium thereby obtained is measured,

4) the concentration of the antigen to be assayed is deduced by reference to standards.

**Patentansprüche**

**1.** Immunologisches Bestimmungsverfahren bei der Fluoreszenzpolarisation, dadurch gekennzeichnet, daß das verwendete Bestimmungsreagens ein 4-Aminonaphtalimid-Derivat der Formel I ist:

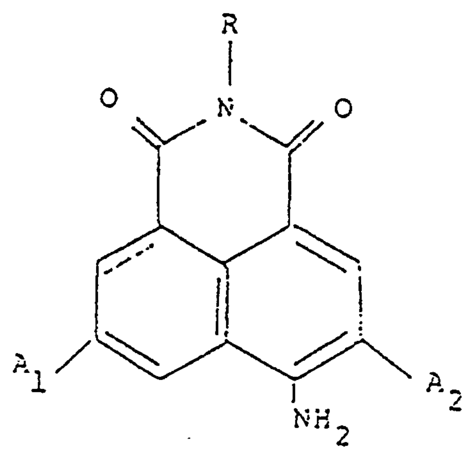

worin

- $A_1$ und $A_2$, die identisch oder verschieden sein können, ein Wasserstoff-Atom oder eine Gruppe $SO_3^- M^+$ bedeuten, worin $M^+$ für ein Alkali- oder Ammonium-Kation steht, und
- R mit dem daran gebundenen Stickstoff-Atom für eine Gruppe steht, die abgeleitet ist von einem

Hapten, einem Antigen mit einem Molekulargewicht von unter 20 000, oder einem Antikörper-Fragment.

2. Verfahren nach Anspruch 1, dadurch gekennzeichnet, daß $A_1$ und $A_2$ in Formel I gleichzeitig für $SO_3^-$ $M^+$ stehen, worin $M^+$ für ein Alkali- oder Ammonium-Kation steht.

3. Verfahren nach Anspruch 1, dadurch gekennzeichnet, daß R in Formel I durch Reaktion eines Haptens, eines Antigens oder eines Antikörper-Fragments mit einer Verbindung der Formel II entsteht:

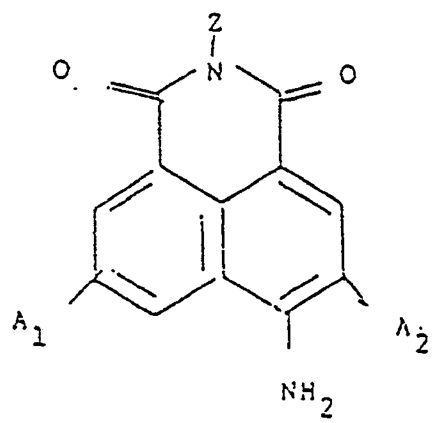

II

worin $A_1$ und $A_2$ die in Anspruch 1 angegebenen Bedeutungen haben, und Z eine Gruppe ist, die mit dem Hapten, dem Antigen oder dem Antikörper-Fragment reagieren kann, ohne die immunologischen Eigenschaften des Haptens, Antigens oder Antikörper-Fragments zu verändern.

4. Verfahren nach Anspruch 3, dadurch gekennzeichnet, daß Z in Formel II ausgewählt ist aus

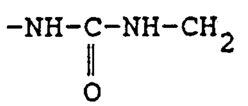

und

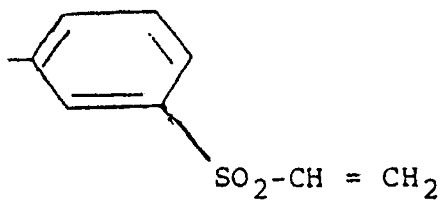

5. Verfahren nach einem der Ansprüche 1 bis 4, wobei das Hapten, das Antigen oder das Antikörper-Fragment über den Imid-Stickstoff gebunden ist oder indirekt über Z, d.h., über eine bifunktionelle Zwischengruppe.

6. Verfahren nach Anspruch 1, dadurch gekennzeichnet, daß das verwendete Reagens entsteht durch Einwirkung eines Haptens, eines Antigens oder eines Antikörper-Fragments mit primärer Aminofunktion, oder eines ihrer Derivate mit primärer Aminofunktion auf das Anhydrid der Formel III:

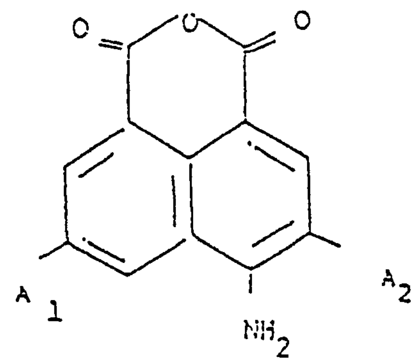

III

worin $A_1$ und $A_2$ die in Anspruch 1 angegebenen Bedeutungen haben.

7. Fluoreszierende Verbindung, verwendbar zur immunologischen Bestimmung bei der Fluoreszenzpolarisation, nach irgendeinem der Ansprüche 1 bis 6, die ein 4-Aminonaphtalimid-Derivat der Formel I ist ;

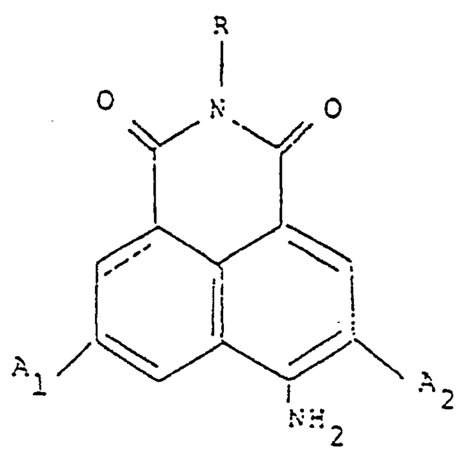

I

worin

- $A_1$ und $A_2$, die identisch oder verschieden sein können, ein Wasserstoff-Atom oder eine Gruppe $SO_3^- M^+$ bedeuten, worin $M^+$ für ein Alkali- oder Ammonium-Kation steht, und
- R mit dem daran gebundenen Stickstoff-Atom für eine Gruppe steht, die abgeleitet ist von einem Hapten, einem Antigen mit einem Molekulargewicht von unter 20 000, oder einem Antikörper-Fragment, und durch Reaktion eines Haptens, eines Antigens oder eines Antikörper-Fragments mit einer Verbindung der Formel II entstehen kann:

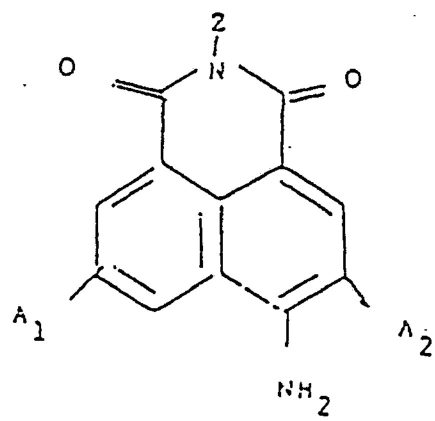

worin $A_1$ und $A_2$ die durch die Verbindung der Formel I angegebenen Bedeutungen haben, und Z eine Gruppe ist, die mit dem Hapten, dem Antigen oder dem Antikörper-Fragment reagieren kann, ohne die immunologischen Eigenschaften des Haptens, Antigens oder Antikörper-Fragments zu verändern, wobei das Reagens so beschaffen ist, daß das Antigen oder das Antikörper-Fragment über den Imid-Stickstoff oder indirekt über Z gebunden ist, und das Hapten über Z indirekt gebunden ist, d.h., über eine vermittelnde bifunktionelle Gruppe, dadurch gekennzeichnet, daß die bifunktionelle Gruppe ausgewählt ist aus aliphatischen Diaminen, γ-Aminobuttersäure oder Cysteamin.

**8.** Verbindung nach Anspruch 7, worin ein Derivat des Haptens oder das zu bestimmende Antigen an die fluoreszierende Ausgangsverbindung gebunden ist.

**9.** Verbindung nach Anspruch 8, worin das Hapten-Derivat ein Steroid ist, worin ein Carbonyl zu der Funktion $-C=N-O-CH_2COOH$ umgesetzt wurde, oder worin ein Hydroxyl zu der Funktion $OCO(CH_2)_2COOH$ umgesetzt wurde.

**10.** Verfahren zur Bestimmung von Haptenen oder Antigenen mit geringem Molekulargewicht durch Fluoreszenz-Immunpolarisation nach irgendeinem der Ansprüche 1 bis 6, dadurch gekennzeichnet, daß

1) in das zu untersuchende Medium eine bekannte Menge eines Reagens der Formel I eingebracht wird, das vom zu bestimmenden Produkt abgeleitet ist und vom gleichen Antikörper wie das zu bestimmende Produkt erkannt wird;

2) eine gegebene Menge an Antikörpern eingebracht wird, die gegen das zu bestimmende Produkt und die vorher zugesetzte Verbindung gerichtet sind ;

3) nach der Inkubation die Höhe der Fluoreszenzpolarisation der so behandelten Probe gemessen wird;

4) die Konzentration des zu bestimmenden Produkts im Medium durch Vergleich mit Eichproben bestimmt wird.

**11.** Verfahren zur Bestimmung von Antigenen mit hohem Molekulargewicht durch Fluoreszenz-Immunpolarisation nach irgendeinem der Ansprüche 1 bis 6, dadurch gekennzeichnet, daß

1) in das zu untersuchende Medium eine bekannte Menge einer fluoreszierenden Verbindung der Formel I eingebracht wird, die ein Fragment eines monoklonalen Antikörpers trägt, der gegen das zu bestimmende Antigen gerichtet ist, wobei dieses Fragment das Antigen erkennt;

2) die Mischung inkubiert wird;

3) die Höhe P der Fluoreszenzpolarisation des so erhaltenen Mediums gemessen wird;

4) die Konzentration des zu bestimmenden Antigens durch Vergleich mit Eichproben ermittelt wird.

FIG.1

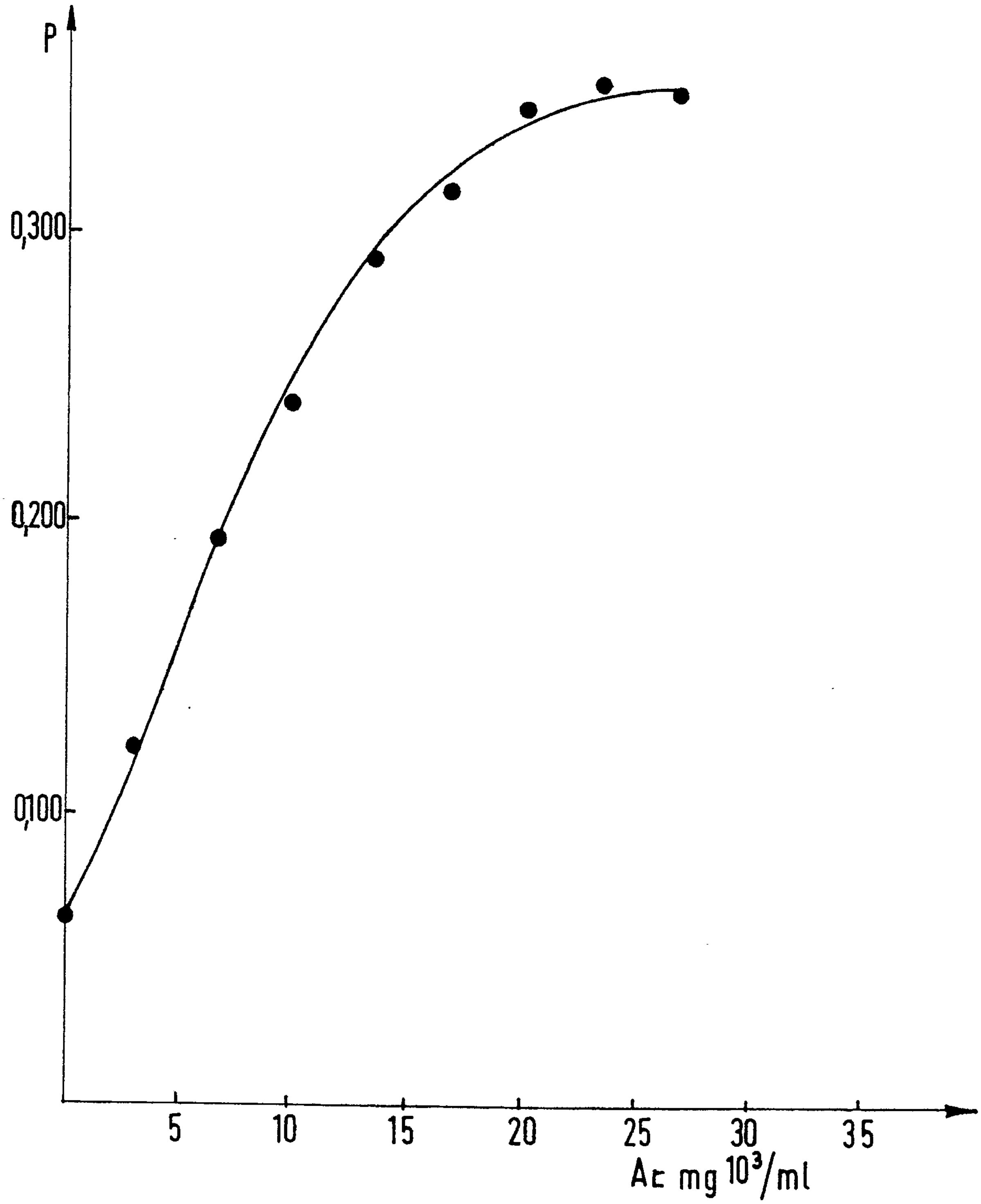

FIG. 2